(19) Europäisches Patentamt
European Patent Office
Office européen des brevets

(11) Veröffentlichungsnummer: **0 129 765**
**A2**

(12) # EUROPÄISCHE PATENTANMELDUNG

(21) Anmeldenummer: **84106659.0**

(22) Anmeldetag: **12.06.84**

(51) Int. Cl.[4]: **C 09 B 57/04**
D 06 P 3/04, C 07 D 403/04
C 07 D 403/14, C 07 D 417/14
C 07 D 401/14

(30) Priorität: **23.06.83 DE 3322666**

(43) Veröffentlichungstag der Anmeldung:
**02.01.85 Patentblatt 85/1**

(84) Benannte Vertragsstaaten:
**CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Rolf, Meinhard, Dr.**
**Berta-von-Suttner-Strasse 24**
**D-5090 Leverkusen 1(DE)**

(72) Erfinder: **Neeff, Rütger, Dr.**
**Berta-von-Suttner-Strasse 22**
**D-5090 Leverkusen 1(DE)**

(54) **Isoindolverbindungen sowie deren Herstellung und Verwendung zum Färben von Polyamiden.**

(57) Verbindungen der Formel

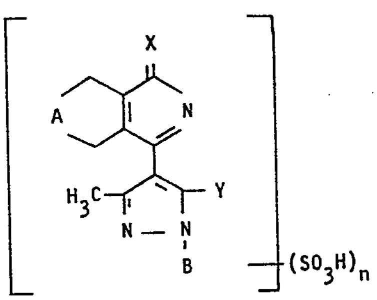

worin

A die restlichen Glieder eines gegebenenfalls substituierten aromatisch carbocyclischen oder heterocyclischen Ringes;

B Wasserstoff oder einen gegebenenfalls substituierten aromatisch carbocyclischen Rest und

X einen zweiwertigen Rest einer Verbindung mit 2 austauschbaren Wasserstoffatomen an einem C- oder N-Atom, insbesondere einen zweiwertigen Rest einer methylenaktiven Verbindung, eines vorzugsweise aromatischen Amins, Hydrazins, Hydrazids oder Hydrazons;

Y die Gruppen OH oder vorzugsweise $NH_2$,

n die Zahlen 1 oder 2

bedeuten,

eignen sich hervorragend zum Färben von synthetischen und natürlichen Polyamiden in gelben und roten Tönen. Insbesondere die Färbungen auf Wolle zeichnen sich durch eine gute Lichtechtheit aus.

EP 0 129 765 A2

BAYER AKTIENGESELLSCHAFT 5090 Leverkusen, Bayerwerk

K/m-c

Isoindolverbindungen sowie deren Herstellung und Verwendung zum Färben von Polyamiden

Die Erfindung betrifft sulfosäuregruppenhaltige Isoindolverbindungen, die in einer ihrer tautomeren Formen der Formel

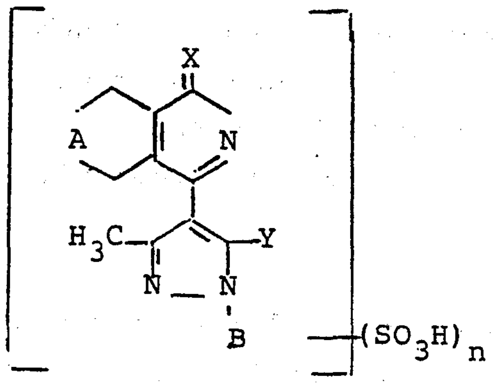

(I)

entsprechen.

In der Formel (I) bedeuten:

A die restlichen Glieder eines gegebenenfalls substituierten aromatisch carbocyclischen oder heterocyclischen Ringes;

B Wasserstoff oder einen gegebenenfalls substituierten aromatisch carbocyclischen Rest und

X einen zweiwertigen Rest einer Verbindung mit 2 austauschbaren Wasserstoffatomen an einem C- oder N-Atom, insbesondere einen zweiwertigen Rest einer methylenaktiven Verbindung, eines vorzugsweise aromatischen Amins, Hydrazins, Hydrazids oder Hydrazons;

Y die Gruppen OH oder vorzugsweise $NH_2$,

n die Zahlen 1 oder 2.

Vorzugsweise steht A für den Rest eines Benzol- oder Naphthalinringes, der beispielsweise 1 - 4 Substituenten aus der Reihe Halogen - insbesondere Chlor und Brom; $C_1$-$C_6$-Alkyl, insbesondere Methyl und Ethyl; $C_1$-$C_6$-Alkoxy, insbesondere Methoxy und Ethoxy; Carboxyl; Nitro oder Carbamoyl tragen kann.

Beispielhaft für die restlichen Glieder eines heterocyclischen Ringes A sei der Rest der Formel

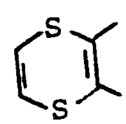

(II)

genannt.

Der zweiwertige Rest X in der Formel (I) kann abgeleitet sein von einer methylenaktiven Verbindung der Formel (III)

$$NC - CH_2 - R \qquad (III)$$

worin R einen die Methylengruppe aktivierenden, d.h. elektronenziehenden Rest bedeutet, beispielsweise Cyan; gegebenenfalls durch $C_1$-$C_6$-Alkyl, Benzyl, Naphthyl oder Phenyl substituiertes Carbamoyl, wobei Phenyl, Benzyl, Naphthyl, z.B. durch Chlor, Brom, $C_1$-$C_6$-Alkyl, $C_1$-$C_6$-Alkoxy, Nitro, Trifluormethyl, $C_1$-$C_6$-Alkylcarbonyl, insbesondere Acetyl, Cyan, $C_1$-$C_6$-Alkylamino, Benzoylamino, Phthalimidyl, Carbamoyl, substituiert sein können; $C_1$-$C_6$-Alkylcarbonyl, insbesondere Acetyl; Benzoyl; $C_1$-$C_6$-Alkoxycarbonyl; Benzyloxycarbonyl; Phenoxycarbonyl, wobei Benzoyl, Benzyl und Phenoxy beispielsweise durch Halogen, wie Chlor und Brom, Nitro, $C_1$-$C_6$-Alkyl, Acylamino, insbesondere $C_1$-$C_6$-Alkylcarbonylamino, Phthalimidyl, substituiert sein können; gegebenenfalls durch Halogen, insbesondere Chlor und Brom, Nitro, Cyan, Trifluormethyl substituiertes Phenyl.

R kann weiterhin für einen heterocyclischen Rest der Formel

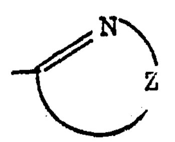

(IV)

stehen, in dem Z für die restlichen Glieder eines gegegebenenfalls weitere Heteroatome enthaltenden 5- oder 6-gliedrigen Ringes steht. Beispiele für heterocyclische Reste R sind:

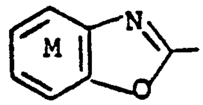 (V)

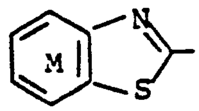 (VI)

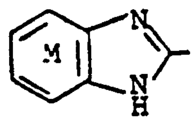 (VII)

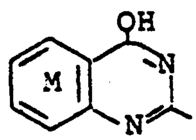 (VIII)

Die mit M bezeichneten Ringe in den Formeln (V) - (VIII) können substituiert sein, z.B. durch Halogen, vorzugsweise Chlor und Brom; Nitro; $C_1$-$C_6$-Alkyl, vorzugsweise Methyl und Ethyl; $C_1$-$C_6$-Alkoxy, vorzugsweise Methoxy und Ethoxy.

Weiterhin kann der Rest X von cyclischen methylenaktiven Verbindungen beispielsweise der folgenden Formeln abgeleitet sein:

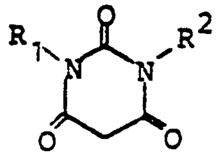 (IX)

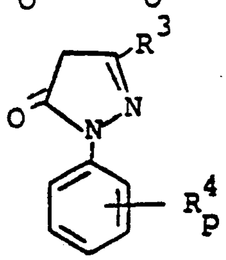 (X)

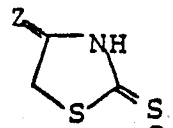

(XI)

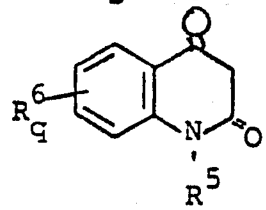

(XII)

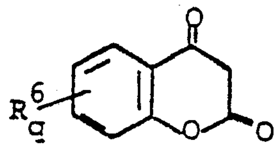

(XIII)

In den Formeln (IX) - (XIII) bezeichnen z.B.:

$R_1$, $R^2$ Wasserstoff; $C_1$-$C_6$-Alkyl; gegebenenfalls durch Halogen, wie Chlor und Brom, $C_1$-$C_6$-Alkyl, $C_1$-$C_6$-Alkoxy oder Nitro substituiertes Phenyl; - und ß-Naphthyl;

$R^3$ $C_1$-$C_6$-Alkyl, vorzugsweise Methyl; Amino; $C_1$-$C_6$-Alkylcarbonyl; Carbamoyl; $C_1$-$C_6$-Alkoxycarbonyl;

$R^4$ $C_1$-$C_6$-Alkyl; $C_1$-$C_6$-Alkoxy; Halogen, vorzugsweise Chlor; Nitro;

p 0, 1 oder 2;

Z 0 oder S;

$R^5$ Wasserstoff oder $C_1$-$C_6$-Alkyl, vorzugsweise Methyl;

$R^6$ Halogen, vorzugsweise Chlor, Nitro oder $CH_3$;

q 0, 1, 2, 3 oder 4.

Steht X für den Rest eines Amins, so handelt es sich vorzugsweise um ein Amin der Formel

$$R^7 - NH_2 \qquad (XIV),$$

in der

$R^7$ für einen gegebenenfalls maximal 3-fach durch Halogen, wie Chlor und Brom, Nitro, Cyan, Carbamoyl, Trifluormethyl, Phthalimidyl, $C_1$-$C_6$-Alkylcarbonylamino, vorzugsweise Acetylamino, Benzoylamino, das seinerseits durch Chlor, $C_1$-$C_6$-Alkyl, vorzugsweise Methyl oder Nitro weitersubstituiert sein kann, substituierten Phenylrest; α- oder ß-Naphthyl oder einen Rest der Formeln

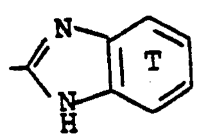

(XV)

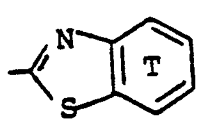

(XVI)

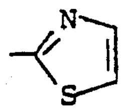

(XVII)

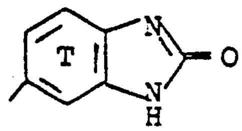

(XVIII)

steht, wobei die mit T bezeichneten Ringe substituiert sein können, z.B. durch Chlor, Brom, Nitro, Methyl, Ethyl, Methoxy oder Ethoxy.

Geeignete Hydrazinreste X leiten sich vorzugsweise von Hydrazinen der Formel

$$R^7 - NH - NH_2 \qquad (XIX)$$

ab, in der

$R^7$ die zu Formel (XIV) angegebene Bedeutung hat.

X in Formel (I) steht weiterhin für ein Hydrazid der Formel

$$R^8 - NH - NH_2 \qquad (XX)$$

in der

$R^8$ vorzugsweisweise $C_1$-$C_6$-Alkylcarbonyl oder gegegebenenfalls durch Halogen, wie Chlor und

Brom, Nitro, Cyan, Carbamoyl, $C_1$-$C_6$-Alkylcarbonylamino, vorzugsweise Acetylamino, Benzoylamino, Phthalimidyl substituiertes Benzoyl bezeichnet.

Schließlich kann sich X von einem Hydrazon der Formel

$$R^9 - \overset{R^{10}}{\overset{|}{C}} = N - NH_2 \qquad (XXI)$$

ableiten, in der

$R^9$ vorzugsweise für Wasserstoff oder $C_1$-$C_6$-Alkyl und

$R^{10}$ vorzugsweise für gegebenenfalls durch Chlor, Nitro, $C_1$-$C_6$-Alkyl, $C_1$-$C_6$-Alkylcarbonylamino, vorzugsweise Acetylamino, Phthalimidyl, Cyan, Carbamoyl oder Trifluormethyl substituiertes Phenyl oder einen heterocyclischen Rest der Formeln

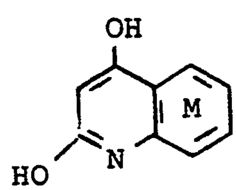

(XXII)

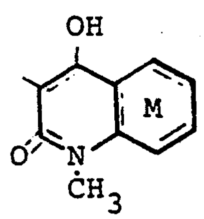

(XXII a)

in denen M die oben angegebene Bedeutung hat, stehen.

Als Substituenten für den aromatischen Rest B in Formel (I) kommen infrage 1 oder 2 Sulfonsäuregruppen und 0 - 4 Substituenten aus der Reihe Halogen, insbesondere Chlor

und Brom; $C_1$-$C_6$-Alkyl, insbesondere Methyl; $C_1$-$C_6$-Alkoxy, insbesondere Methoxy und Ethoxy; Cyan; Nitro; Trifluormethyl.

Bevorzugte Verbindungen der Formel (I) entsprechen in einer ihrer tautomeren Formen der Formel

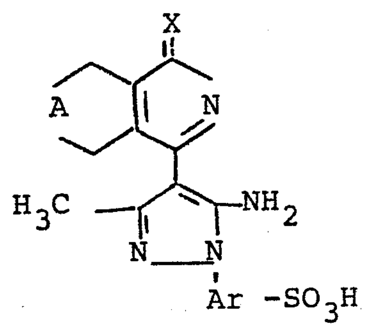

(XXIII)

worin A und X die oben genannte Bedeutung haben und Ar einen Benzol- oder Naphthalinrest bedeutet, der die vorstehend für R genannten Substituenten tragen kann.

Besonders bevorzugte Verbindungen der Formel (I) entsprechen in einer ihrer tautomeren Formen der Formel

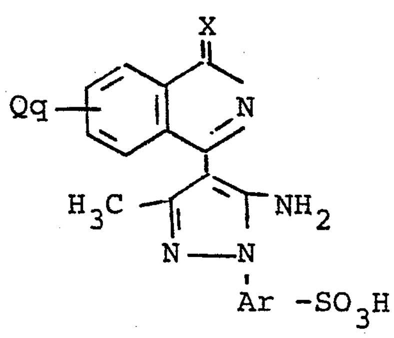

(XXIV)

worin X und Ar die oben genannte Bedeutung haben und Q einen der oben als geeignete Substituenten in A angegebenen Reste bedeutet.

Ganz besonders bevorzugt sind Verbindungen der Formel

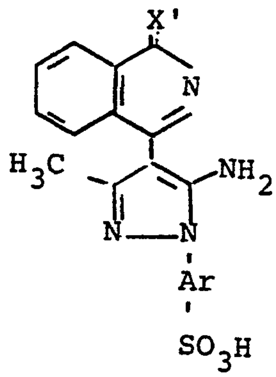

(XXV)

worin

Ar einen gegebenenfalls ein- oder zweifach durch Cl substituierten Phenylen- oder Naphthylenrest und

X' einen Rest der Formeln

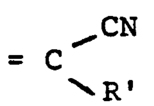, =N-NH-U, =N-NH-CO-U, =N-N=CH-U,

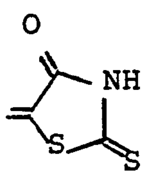, 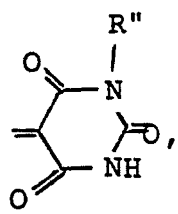, 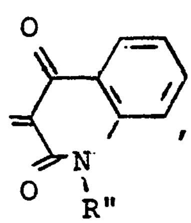, 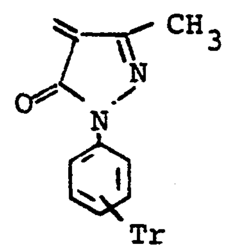,

oder 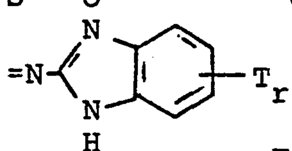 bedeuten, wobei

R' für CONHV, COOW, 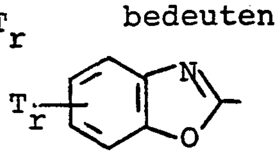,

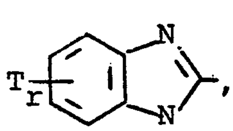, 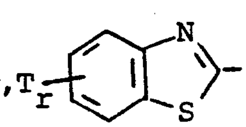 oder 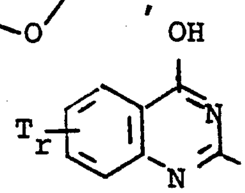,

U für 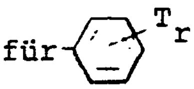

V für H, $C_1$-$C_4$-Alkyl oder U,

W für $C_1$-$C_4$-Alkyl oder U,

T für Cl oder $CH_3$

R" für H oder $CH_3$ und

r für 0, 1 oder 2 stehen.

Hervorzuheben sind weiterhin Verbindungen der Formel XXV mit einer OH-Gruppe an Stelle der $NH_2$-Gruppe.

Die Herstellung der Verbindungen der Formel I kann nach verschiedenen Verfahren erfolgen:

1. Kondensation des Zwischenproduktes (XXVI) mit dem Pyrazolderivat (XXVII)

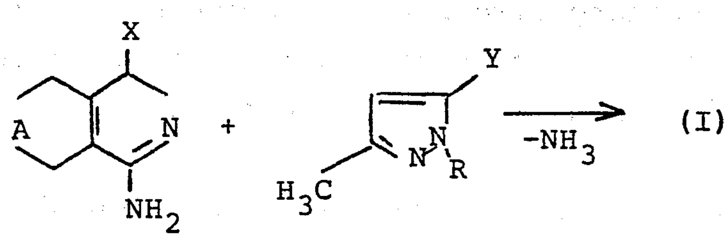

(I)

(XXVI) (XXVII)

Verbindungen des Typs (XXVI) und (XXVII) sind literaturbekannt.

Die Umsetzung erfolgt zweckmäßig unter schwach sauren Bedingungen in Wasser oder einem organischen Lösungsmittel bei Temperaturen zwischen 40 und 120°C.

Als organische Lösungsmittel können verwendet werden Alkohole wie Methanol, Ethanol, n-Butanol; Aromaten wie Chlorbenzol, 1,2-Dichlorbenzol, Nitrobenzol, Toluol oder Xylol; Ethylenglykol und dessen Ether, z.B. Ethylenglykolmonomethylether; amidische Lösungsmittel wie Formamid, Dimethylformamid, N-Methylpyrrolidon; Dimethylsulfoxid oder Tetramethylensulfon.

2. Kondensation des Zwischenproduktes (XXVIII) mit der Komponente $H_2X$ (XXIX)

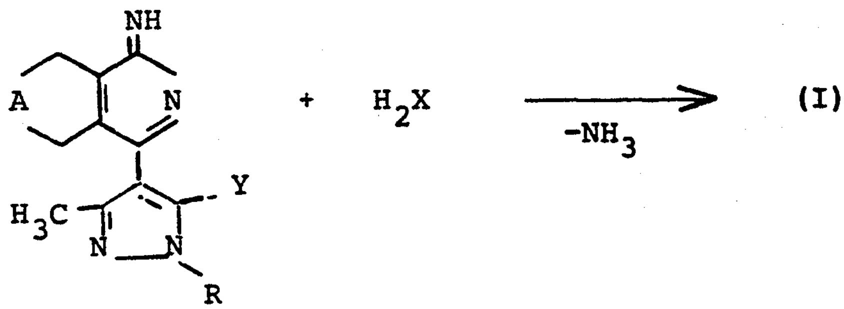


(XXVIII) (XXIX)

Die Umsetzung wird zweckmäßigerweise unter den gleichen Bedingungen durchgeführt, wie sie weiter oben für die Variante 1 genannt wurden.

Verbindungen der Formel (XXIX) sind ebenfalls bekannt, während die Zwischenprodukte der Formel (XXVIII) in einer vorgeschalteten Stufe aus dem Isoindolenin (XXX) mit dem Pyrazolderivat (XXVII) erhalten werden können.

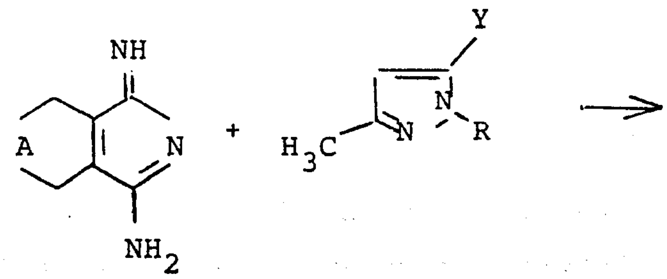 (XXVII)

(XXX) (XXVII)

Die Verbindungen der Formel (I) eignen sich vor allem zum Färben von amidgruppenhaltigen Fasern, die je nach Substitution in roten bis grünstichig gelben Nuancen gefärbt werden, wobei sich insbesondere die Färbungen auf Wolle durch gute Echtheiten, insbesondere Lichtechtheiten, auszeichnen.

Beispiel 1

In 150 ml Wasser gibt man 15 ml Eisessig, 15 g 1-Amino-3-(cyano-N-methylcarbamoylmethylen)-isoindolenin und 17 g 1-(3-Sulfophenyl)-3-methyl-5-pyrazolimin und rührt 60 Min. bei Raumtemperatur und 2 h bei 90°C.

Nach Abkühlen, Absaugen und Waschen erhält man 24 g (80 %) des gelben Farbstoffs der Formel

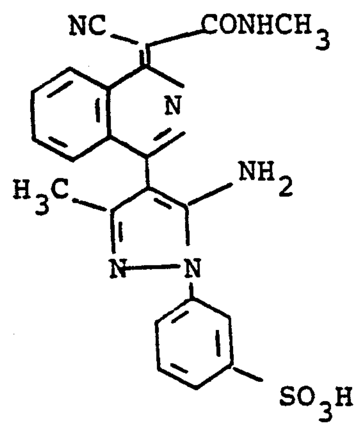

der auf Wolle brilliante grünstichig-gelbe Färbungen mit sehr guter Lichtechtheit liefert.

Beispiel 2

In 200 ml 1,2-Dichlorbenzol gibt man 20 ml Eisessig, 10 g 1-Amino-3-(cyano-N-n-butylcarbamoylmethylen)-isoindolenin und 10 g 1-(3-Sulfophenyl)-3-methyl-5-pyrazolimin und rührt mit Rückflußkühler 3 h bei 120°C.

Nach Abkühlen wird abgesaugt und gewaschen: 14 g (75 %) des gelben Farbstoffs der Formel

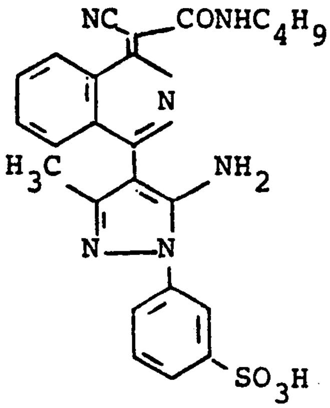

Nach den in Beispiel 1 und 2 genannten Verfahren werden durch Umsetzung der in der Tabelle angegebenen Ausgangsmaterialien mit 1-(3-Sulfophenyl)-3-methyl-5-pyrazolimin die in der Tabelle angeführten Farbstoffe mit den angegebenen Farbtönen auf Wolle erhalten.

| Beispiel | Ausgangsmaterial | Endprodukt | Farbton auf Wolle |
|---|---|---|---|
| 3 | 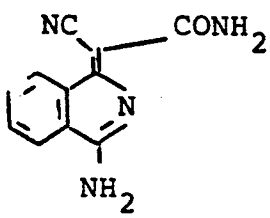 | 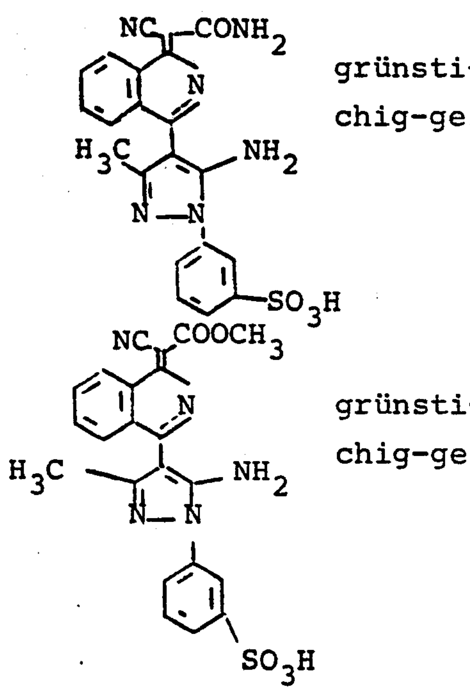 | grünstichig-gelb |
| 4 | 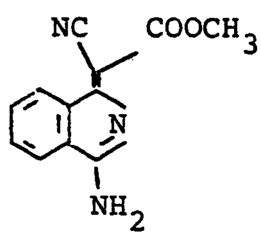 | | grünstichig-gelb |

| Beispiel | Ausgangsmaterial | Endprodukt | Farbton auf Wolle |
|---|---|---|---|
| 5 | 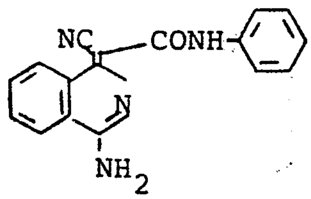 | 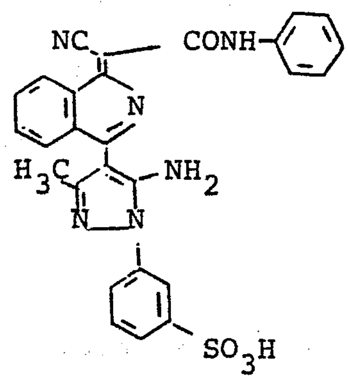 | grünstichig-gelb |
| 6 | 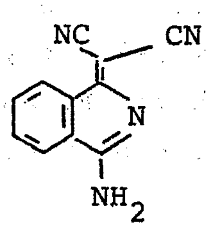 | 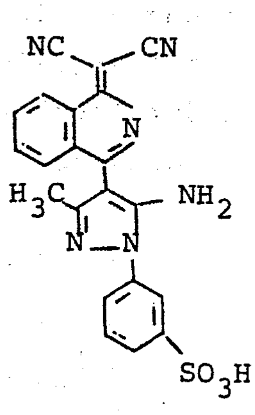 | gelb |
| 7 | 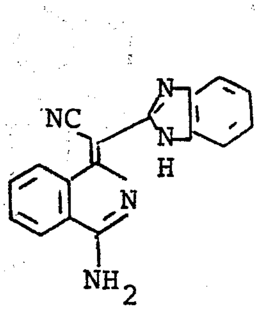 | 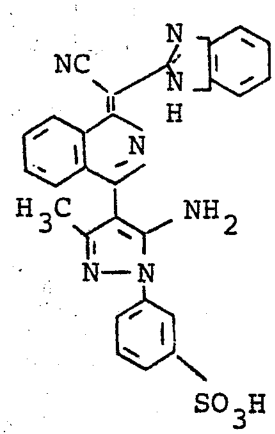 | orange |

| Beispiel | Ausgangsmaterial | Endprodukt | Farbton auf Wolle |
|---|---|---|---|
| 8 | 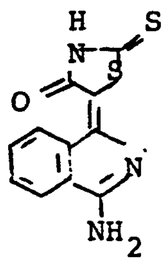 | 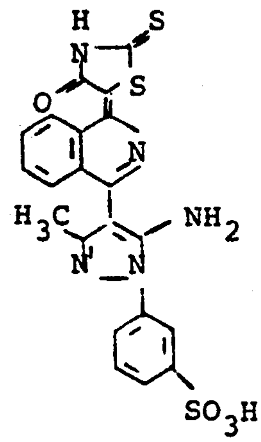 | stumpf-gelb |
| 9 | 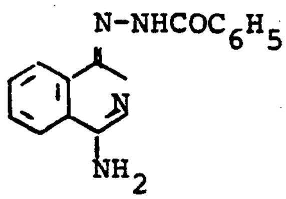 | 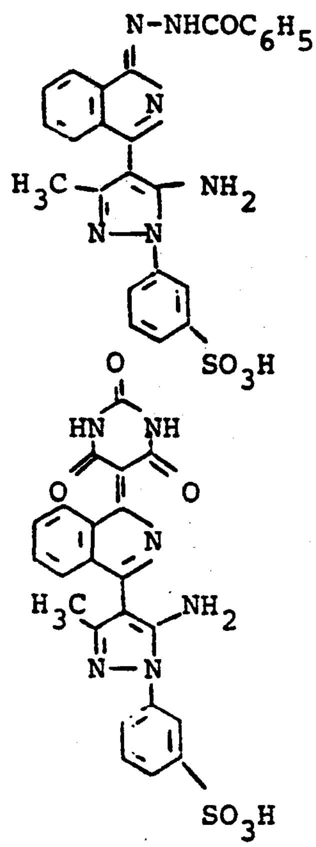 | grünsti-chig-gelb |
| 10 | 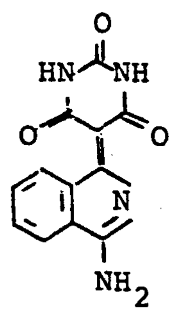 | | gelb |

0129765

| Beispiel | Ausgangsmaterial | Endprodukt | Farbton auf Wolle |
|---|---|---|---|
| 11 | 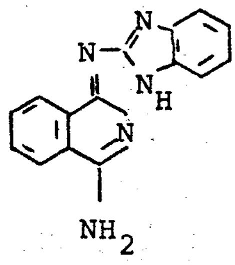 | 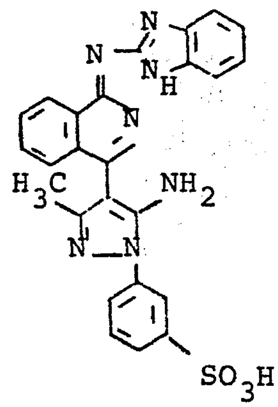 | grünstichig-gelb |
| 12 | 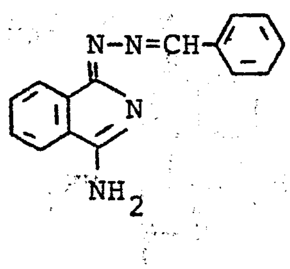 | 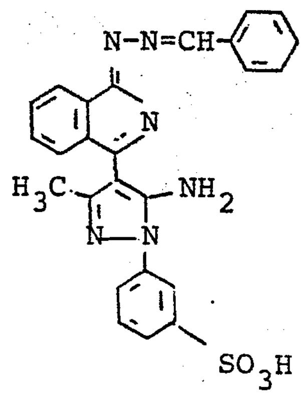 | gelb |
| 13 | 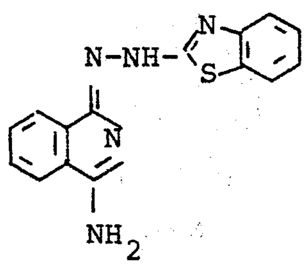 | 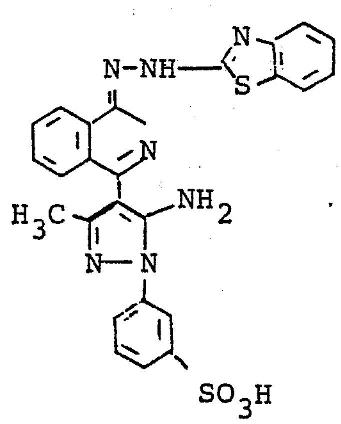 | rotstichig-gelb |

| Beispiel | Ausgangsmaterial | Endprodukt | Farbton auf Wolle |
|---|---|---|---|
| 14 | 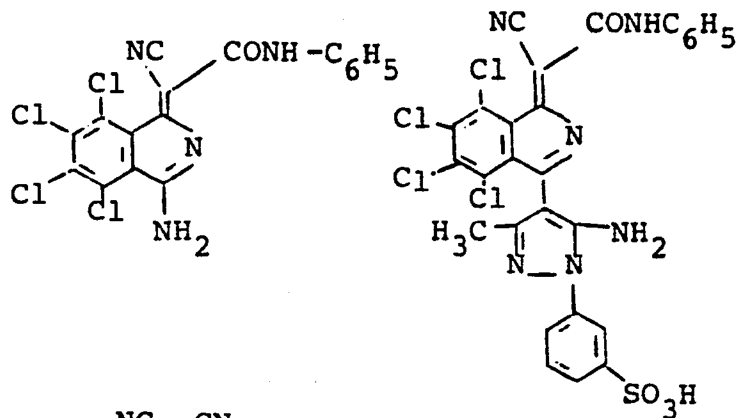 | | gelb |
| 15 | 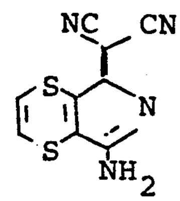 | 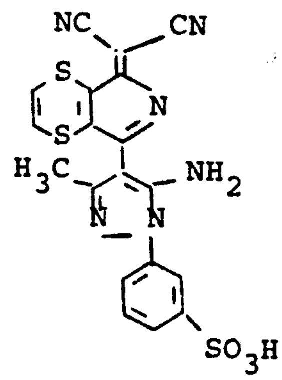 | braun |

Verfährt man nach Beispiel 1 und 2 und verwendet 3-Methyl-1-(8-sulfo-2-naphthyl)-5-pyrazolimin anstelle von 3-Methyl-1-(3-sulfophenyl)-5-pyrazolimin, erhält man mit den in Tabelle 2 angegebenen Ausgangsmaterialien die aufgeführten Woll-Farbstoffe mit den angegebenen Farbtönen.

| Beispiel | Ausgangsmaterial | Endprodukt | Farbton auf Wolle |
|---|---|---|---|
| 16 | 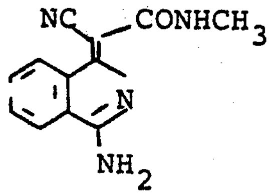 | 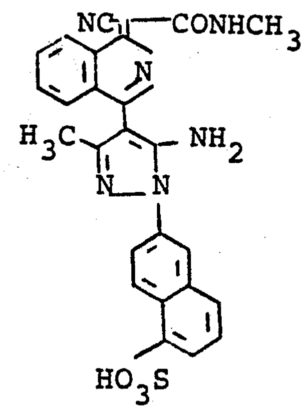 | grünstichig-gelb. |
| 17 | 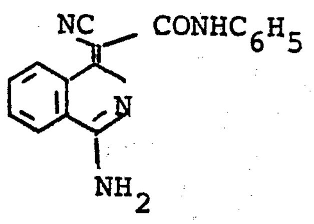 | 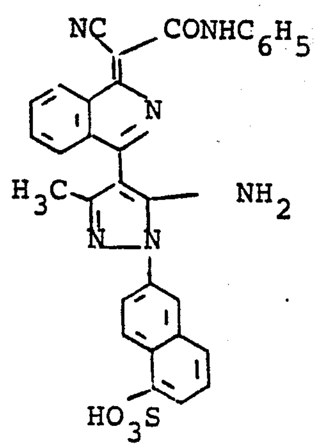 | grünstichig-gelb |
| 18 | 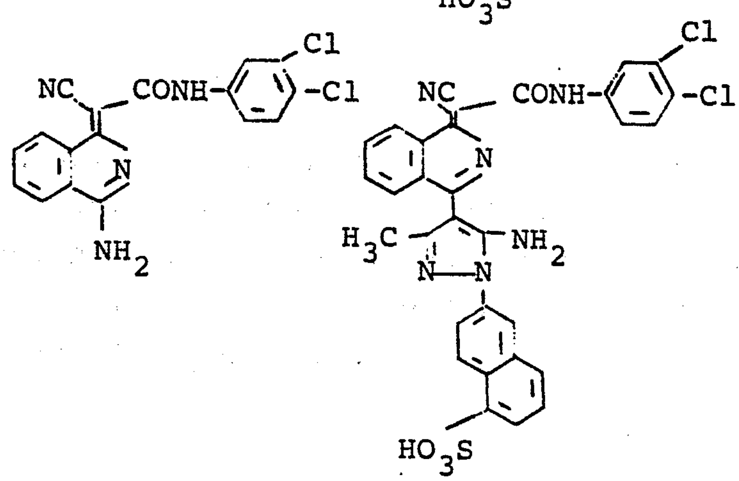 | | grünstichig-gelb |

| Beispiel | Ausgangsmaterial | Endprodukt | Farbton auf Wolle |
|---|---|---|---|
| 19 | 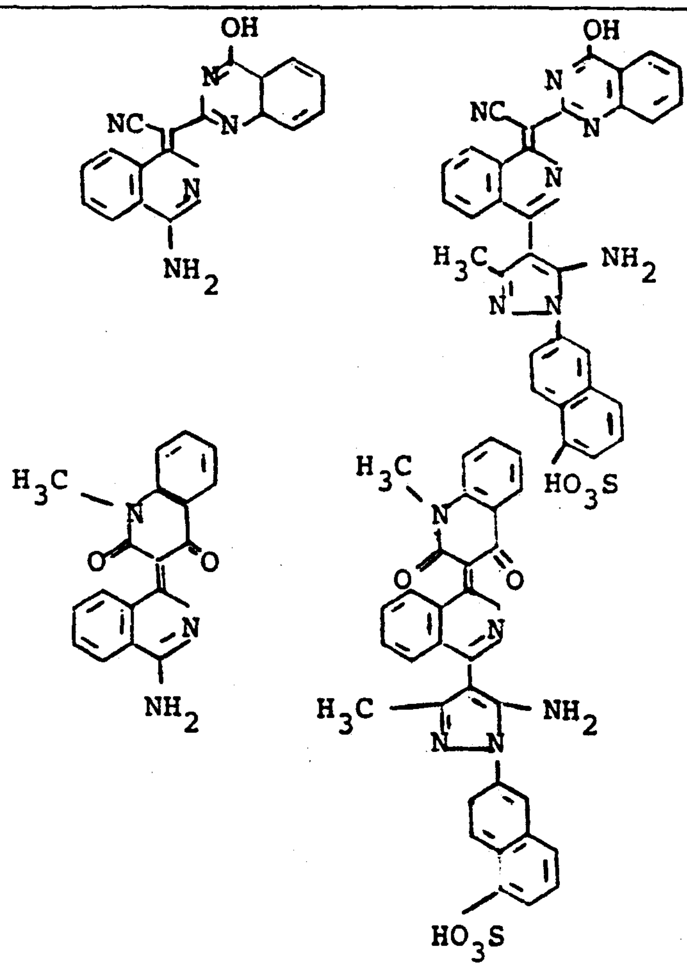 | | grünstichig-gelb |
| 20 | | | grünstichig-gelb |

Beispiel 21

In 150 ml DMF gibt man 10 ml Eisessig, 15 g 1-Amino-(3-cyano-carbamoylmethylen)-isoindolenin und 20 g 1-(4-Sulfophenyl)-3-methyl-5-pyrazolon und rührt 4 h bei 80°C.

Nach Absaugen bei Raumtemperatur und Wäsche verbleiben 22 g (70 %) des Farbstoffs der Formel,

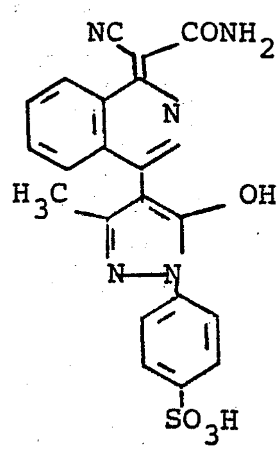

der synthetischen Polyamid-6-fasern in blaustichigen Rottönen anfärbt.

Beispiel 22

Analog zu Beispiel 21 erhält man bei Einsatz von 25,5 g 1-(2,5-Dichlor-4-sulfophenyl)-3-methyl-5-pyrazolon 24 g (65 %) des Farbstoffs der Formel

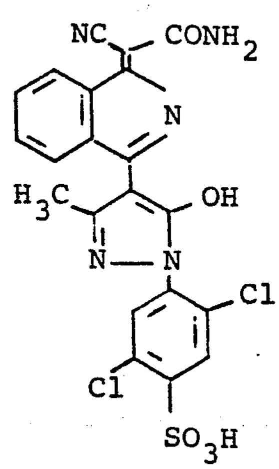

der synthetischen Polyamid-6-fasern in stark blaustichig roten Tönen anfärbt.

Nach dem in Beispiel 21 genannten Verfahren erhält man durch Umsetzung von 1-(4-Sulfophenyl)-3-methyl-5-pyrazolon mit den in Tabelle 2 angegebenen Isoindoleninen die aufgeführten Farbstoffe mit den angegebenen Farbtönen.

| Beispiel | Ausgangsmaterial | Endprodukt | Farbton auf Polyamid-6 |
|---|---|---|---|
| 23 | 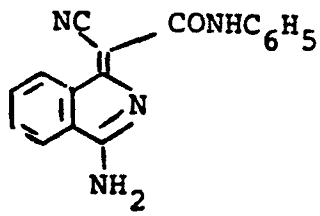 | 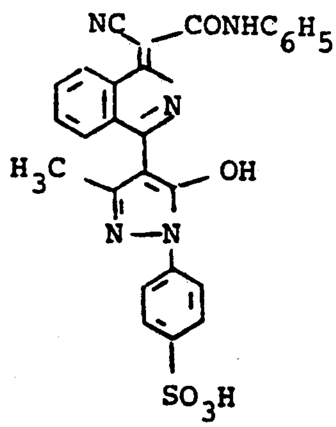 | rot |
| 24 | 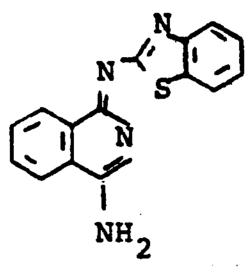 | 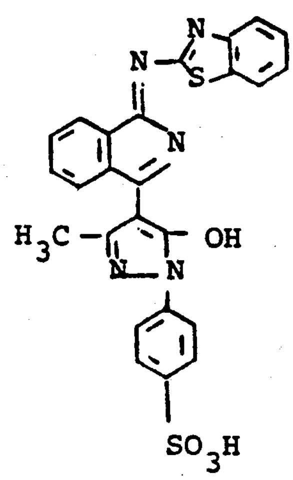 | rot |

| Beispiel | Ausgangsmaterial | Endprodukt | Farbton auf Poly-amid-6 |
| --- | --- | --- | --- |
| 25 | 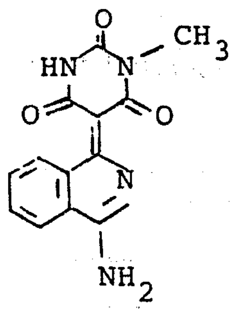 | 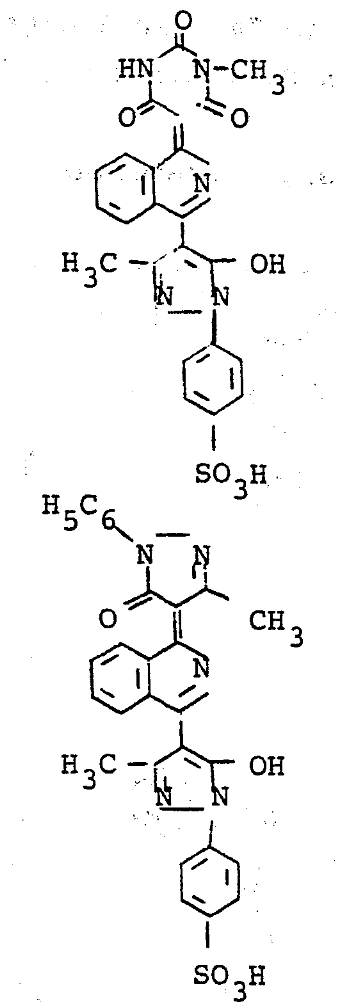 | rot |
| 26 | 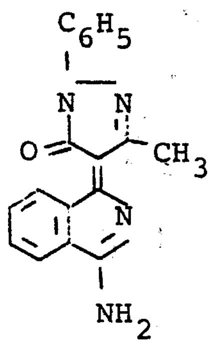 | | braun |

Patentansprüche:

1. Verbindungen, die in einer ihrer tautomeren Formen der Formel

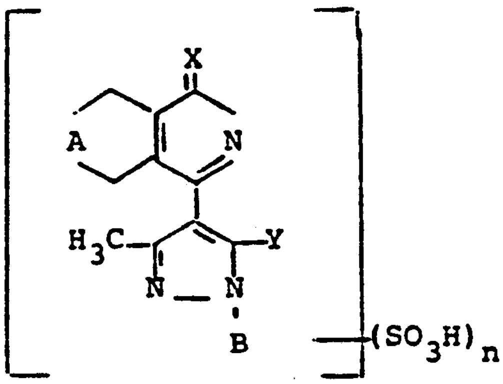

(I)

entsprechen, worin

A die restlichen Glieder eines gegebenenfalls substituierten aromatisch carbocyclischen oder heterocyclischen Ringes;

B Wasserstoff oder einen gegebenenfalls substituierten aromatisch carbocyclischen Rest und

X einen zweiwertigen Rest einer Verbindung mit 2 austauschbaren Wasserstoffatomen an einem C- oder N-Atom, insbesondere einen zweiwertigen Rest einer methylenaktiven Verbindung, eines vorzugsweise aromatischen Amins, Hydrazins, Hydrazids oder Hydrazons;

Y die Gruppen OH oder vorzugsweise $NH_2$ und

n die Zahlen 1 oder 2

bedeuten.

2. Verbindungen gemäß Anspruch 1 der Formel

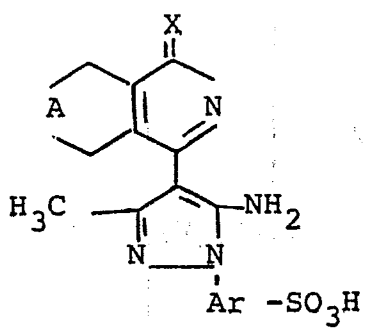

(XXIII)

worin

Ar für einen gegebenenfalls durch Cl, Br, $C_1$-$C_6$-Alkyl, $C_1$-$C_6$-Alkoxy, $CF_3$, $NO_2$ oder CN substituierten Benzol- oder Naphthalinrest steht und die übrigen Reste die in Anspruch 1 genannte Bedeutung haben.

3. Verbindungen gemäß Anspruch 1 der Formel

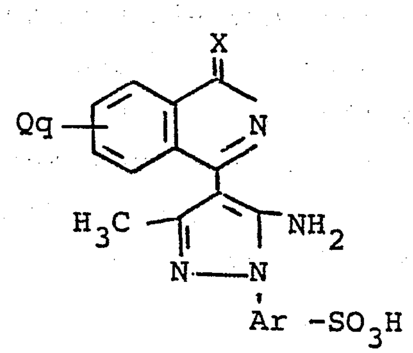

(XXIV)

worin Q für $C_1$-$C_6$-Alkyl, $C_1$-$C_6$-Alkoxy, Cl, Br, COOH, $CONH_2$ oder $NO_2$ und q für 0-4 stehen und die übrigen Reste die in den Ansprüchen 1-2 genannten Bedeutungen haben.

4. Verbindungen gemäß Anspruch 1 der Formel

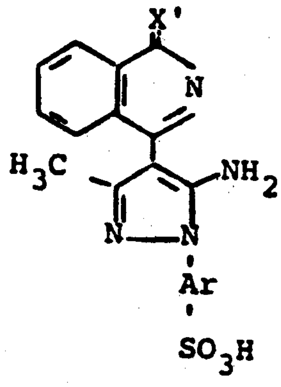

(XXV)

worin

Ar einen gegebenenfalls ein- oder zweifach durch Cl substituierten Phenylen- oder Naphthylenrest und

X' einen Rest der Formeln

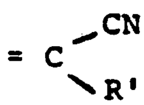 , =N-NH-U, =N-NH-CO-U, =N-N=CH-U,

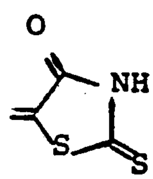 , 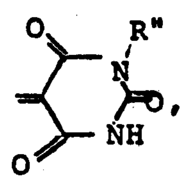 , 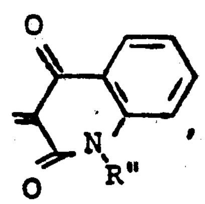 , 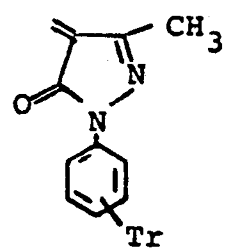 ,

oder 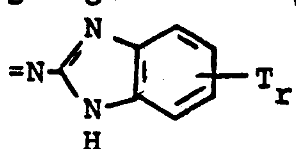 bedeuten, wobei

R' für CONHV, COOW, 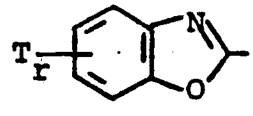 ,

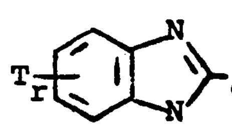 , 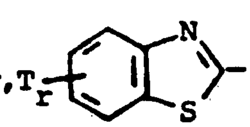 oder 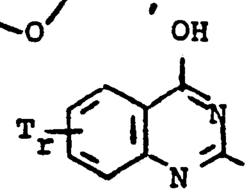 ,

U für 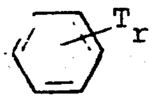

V für H, $C_1$-$C_4$-Alkyl oder U,

W für $C_1$-$C_4$-Alkyl oder U,

T für Cl oder $CH_3$ und

R" für H oder $CH_3$

r für 0, 1 oder 2 stehen.

5. Verbindungen gemäß Anspruch 1, dadurch gekennzeichnet, daß

X für den Rest = 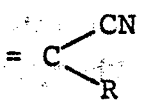 steht,

worin R einen elektronenziehenden Rest bedeutet.

6. Verbindung gemäß Anspruch 1 der Formel

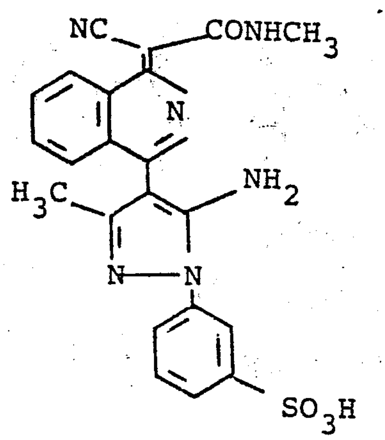

7. Verbindung gemäß Anspruch 1 der Formel

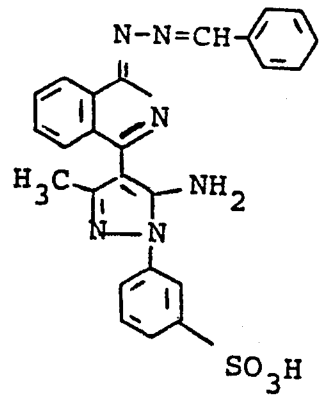

8. Verfahren zur Herstellung von Verbindungen gemäß Anspruch 1, dadurch gekennzeichnet, daß man entweder Verbindungen der Formel

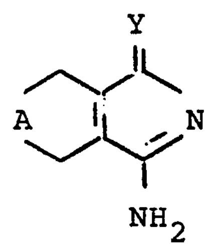

mit Verbindungen der Formel

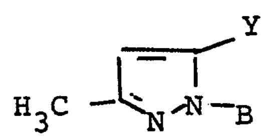

oder Verbindungen der Formel

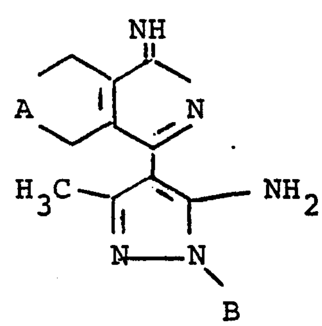

mit Verbindungen der Formel

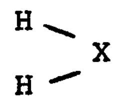

umsetzt.

9. Verwendung der Verbindungen gemäß Anspruch 1 zum Färben von Polyamiden, insbesondere Wolle.